# EUROPEAN PATENT APPLICATION

(11) **EP 4 374 903 A1**
(43) Date of publication of application: **29.05.2024**
(21) Application number: 22209377.5
(22) Date of filing: 24.11.2022
(51) Int. Cl.: A61M 16/14

(54) **ADMINISTERING AN AEROSOL TO A PATIENT**

(71) Applicant: Fraunhofer-Gesellschaft zur Förderung der angewandten Forschung e.V., 80686 München (DE)
(72) Inventor: Wiegandt, Felix, 30625 Hannover (DE); Pohlmann, Gerhard, 31715 Meerbeck (DE)
(74) Representative: Altmann Stößel Dick Patentanwälte PartG mbB

(57) **Abstract**

The present invention relates to an apparatus (110) for administering an aerosol to a patient (116) as well as a processing device (120) configured for controlling the apparatus (110) for administering the aerosol to the patient (116). Herein, the processing device (120) is configured for controlling the apparatus (110) for administering the aerosol (112) to the patient (116) by
- receiving input data related to a respiration pattern of a patient (116), wherein the respiration pattern comprises information about a temporal course of at least one of an elevation (132) or a constriction (134) of at least one of a chest (136) or an abdomen (138) of a patient (116);
- determining at least one point in time from the respiration pattern; and
- controlling an aerosol stream (114) to the patient (116) triggered at the at least one point in time,
wherein the at least one point in time advances at least one of an onset or a suspension of a breathing of the patient (116).

In particular, the apparatus (110) is designed for providing a high aerosol concentration to the patient (116), especially to a preterm infant.

## Description

### Field of the invention

The present invention relates to an apparatus and a method for administering an aerosol to a patient and to a processing device configured for controlling the apparatus for administering the aerosol to the patient. In particular, the apparatus and the method may be designed for providing a defined aerosol concentration to the patient, especially but not limited to a preterm infant. Further applications are feasible.

### Related art

Pulmonary diseases, such as asthma or chronic obstructive pulmonary disease (COPD), are, usually, treated by inhalation of corresponding aerosols. Further, efforts exist to deliver a surfactant in form of an aerosol during ventilation to a preterm infant having a surfactant deficiency, especially since this form of therapy is non-invasive and, thus, less detrimental for the preterm infant in contrast to the standard therapy using invasive instillation of a surfactant suspension.

Aerosols may be continuously generated and administered to a patient without breath synchronized aerosol release. However, a share of a continuously delivered aerosolized drug may be lost and may, thus, show no medical benefit. As further demonstrated by Longest, P.W.; Azimi, M.; Hindle, M., Optimal Delivery of Aerosols to Infants During Mechanical Ventilation, Journal of Aerosol Medicine and Pulmonary Drug Delivery 2014, 27, 371-385, doi:10.1089/jamp.2013.1077, only aerosol delivered during the first half of an inhalation phase reaches the periphery of the alveoli. Further, a local pulmonary drug requirement for an inhaled application of a surfactant considerably may exceed that of other inhaled application of aerosols for pulmonary diseases.

Consequently, a breath-triggered drug release is of high interest. When breath is detected, the aerosol must be released accordingly, i.e. over a defined period of time, such as a short or long aerosol boli. However, known approaches for breathing detecting during respiratory support or independent breathing and for aerosol release offer various problems and shortcomings.

Known respiration monitoring devices work via volume stream or pressure sensors, which are configured to determine an inhalation flow at the patient interface. As a result, a drug release can only occur after with a delay, i.e. not directly at the start of inhalation. Accordingly, a pressurized metered-dose inhaler can be integrated in combination with a spacer into a ventilation circuit. However, a pharmaceutical preparation is only released via active actuation of the inhaler by the patient or a further person. As an alternative, an aerosol generating device can be coupled into the ventilation circuit by using an adapter.

Triggering of the aerosol release can be based on a detection of a pressure change in the ventilation tube caused by breathing, whereupon the aerosol generation is activated or deactivated. However, the drug is released only after a corresponding pressure change has been registered which occurs with a delay. As an alternative, the drug release can be controlled by using a pressure measurement at a mouthpiece, wherein an evaluation algorithm may consider an averaging over consecutive breaths.

US 2021/0283345 A1 discloses an aerosol delivery system having an aerosol generator that aerosolizes a fluid for delivery to a patient as a patient inhales. The aerosol delivery system includes a pump coupled to the aerosol generator that pumps the fluid to the aerosol generator, and a breath sensor that emits a signal as the patient breathes. A controller couples to the aerosol generator, the pump, and the breath sensor. In operation, the controller receives the signal from the breath sensor, controls a flow of fluid to the aerosol generator in response to the signal, and controls the aerosol generator to start aerosolizing the fluid before the patient inhales.

WO 2020/243107 A1 discloses a method of delivering aerosolized surfactant to an infant that includes interfacing an aerosolization device with an airway of an infant and aerosolizing, using the aerosolization device, a volume of surfactant into particles having a mass mean aerodynamic diameter of less than about 3 µm at a rate of at least 0.1 ml/min. The surfactant is aerosolized within about 1 to 8 cm from a patient interface. Aerosol is generated for up to approximately 80% or each inspiration. The method also includes delivering the aerosolized surfactant to the infant's airway.

WO 2019/115771 A1 discloses a nebulizer system comprising a nebulizer for assisted breathing devices. The nebulizer comprises a body having a first connection for connecting the nebulizer to the assisted breathing device and a second connection for connecting the nebulizer to a patient, particularly a neonate, wherein the body forms a flow channel from the first connection to the second connection, and a nebulizing device for nebulizing a liquid. The nebulizing device is arranged in the flow channel between the first connection and the second connection. The nebulizer is configured for being adapted to an oral and/or nasal communication element, such as nasal prongs, a nose mask, a face mask or a mouthpiece. Further, the invention relates to a holding system for holding a nebulizer or a nebulizer system.

WO 2020/079055 A1 discloses methods, devices and compositions for use in inhalation therapy of neonates, infants or children, suffering from a disease, optionally a pulmonary disease, such as asthma, by which high amounts of inhaled drugs are directed to the small airways of the peripheral lungs of neonates, infants and children using slow, controlled flow rates and pre-set inhalation volumes. A novel inhalation device tailored for use in neonates, infants and children and adapted to provide said slow, controlled flow rates with a simplified jet nebulizer set-up is disclosed, together with kits comprising the device.

However, most known approaches are based on predictions that are usually inappropriate since a preterm infant shows an irregular breathing pattern, such that the aerosol cannot be generated and released in an optimal fashion. Owing to an inherent dead space volume, such devices are often not suitable for being used with a preterm infant.

US 2019/0247595 A1 discloses a system for respiration-controlled application of aerosol in powder form during artificial respiration or assisted respiration of a patient including an interface contacting the patient's respiratory tract, a unit for generating a respiratory gas flow, at least one inspiration line through gas flow is conducted to the interface, an aerosol generator, at least one aerosol line through which the generated aerosol is conducted from the aerosol generator to the interface, and a respiration sensor that detects the patient's respiration signal. A valve in the at least one aerosol line is controlled based on the detected respiratory signal. An intermediate store for generated aerosol in powder form is arranged between the valve and the aerosol generator. The gas flow has a first pressure that is higher than or equal to ambient pressure and the aerosol has a second pressure that is higher than or equal to the first pressure.

WO 2012/020004 A1 discloses a device for connection of the airways of a patient on ventilatory support with a source of a breathing gas and a source of an aerosol. Said device comprises a contact component which is adapted to be positioned in contact with the airways of the patient and which comprises: one or two tubes each comprising a lumen through which breathing gas and aerosol can be delivered to the airways of the patient, a mixing lumen which is in fluid connection with the lumen or lumens of the tube or the two tubes and having a longitudinal axis extending substantially perpendicularly to longitudinal axis or axes of the lumen or lumens and a port through which aerosol can be introduced into said mixing lumen and arranged such that the aerosol and a breathing gas can be mixed in the mixing lumen.

Wiegandt, F.C.; Biegger, D.; Fast, J.F.; Matusiak, G.; Mazela, J.; Ortmaier, T.; Doll, T.; Dietzel, A.; Bohnhorst, B.; Pohlmann, G., Detection of Breathing Movements of Preterm Neonates by Recording Their Abdominal Movements with a Time-of-Flight Camera, Pharmaceutics 2021, 13, doi:10.3390/pharmaceutics13050721, describe that the initiation of the patient's inspiration needs to be detected in order to deliver an aerosolized drug in a breath-triggered manner. Respiratory sensors, especially when invasive, can be tested on preterm neonates only with great effort due to clinical and ethical hurdles. Therefore, a physiological model is highly desirable to validate these sensors. For developing such a system, abdominal movement data of preterm neonates are required. The authors recorded time sequences of five preterm neonates' abdominal movements with a time-of-flight camera and successfully extracted various breathing patterns and respiratory parameters. Several characteristic breathing patterns, such as forced breathing, sighing, apnea and crying, were identified from the movement data. Respiratory parameters, such as duration of inspiration and expiration, as well as respiratory rate and breathing movement over time, were also extracted. They demonstrated that respiratory parameters of preterm neonates can be determined without contact. Therefore, such a system can be used for breathing detection to provide a trigger signal for breath-triggered drug release systems.

Koch, E.; Dietzel, A, Skin attachable flexible sensor array for respiratory monitoring, Sensors and Actuators A: Physical 2016, 250, 138-144, doi:10.1016/j.sna.2016.09.020, describe a 6 × 6 sensor array for curvature sensing in a format of a thin flexible polyimide foil. The sensor foil is to be used for respiratory monitoring of premature infants by directly attaching it to the skin for measuring the body deformations caused by breathing. Sensor signals shall in future be used not only to trigger the respiration device but also to provide time dependent body surface reconstruction as a diagnostic tool. One single sensor element consists of four gold strain gauges in a Wheatstone bridge configuration. For suppressing sensor response to foil stretching and for increasing bending sensitivity, they introduced a double-sided sensor design with strain gauges on both surfaces of the thin foil leading to a 170% higher sensitivity than a one-sided sensor design. Double sided sensor elements with different orientations are arranged in an alternating pattern across the array allowing to fully and unambiguously determine the bending vector utilizing plausibility considerations on basis of signals from neighboring elements.

### Problem to be solved

It is therefore an objective of the present invention to provide an apparatus and a method for administering an aerosol to a patient and a processing device configured for controlling the apparatus for administering the aerosol to the patient, which at least partially avoid the problems and shortcomings of the known approaches.

It is particularly desirable that the apparatus and the method would be able to administer a large portion of a pharmaceutical preparation comprised by an aerosol to a patient, especially but not limited to a preterm infant, specifically in order to ensure that an envisaged amount of the pharmaceutical preparation may actually be provided to the patient in a desired concentration and may not be diluted and/or distributed elsewhere. It is further desirable to reduce or, preferably, to avoid a delay in administering the pharmaceutical preparation to the patient. It is still further desirable to be able to administer a pharmaceutical preparation to one or more predefined targets in central or peripheral areas of the lung.

### Summary of the invention

This problem is solved by an apparatus and a method for administering an aerosol to a patient and a processing device configured for controlling the apparatus for administering the aerosol to the patient having the features of the independent claims. Preferred embodiments, which can be implemented in isolated fashion or in any arbitrary combination, are disclosed by the dependent claims and throughout the description.

In a first aspect, the present invention refers to a processing device, which is configured for controlling an apparatus for administering an aerosol to a patient by
- receiving input data related to a respiration pattern of a patient, wherein the respiration pattern comprises information about a temporal course of at least one of an elevation or a constriction of at least one of a chest or an abdomen of a patient;
- determining at least one point in time from the respiration pattern; and
- controlling an aerosol stream to the patient triggered at the at least one point in time, wherein the at least one point in time advances at least one of an onset or a suspension of a breathing of the patient.

As used herein, the term "apparatus" refers to a device that comprises a plurality of elements, wherein each element cooperates with at least one further element to administer an aerosol to a patient. In particular, the apparatus can be embodied as a single device, which may comprise at least all elements required for this purpose. As an alternative, at least two elements of the apparatus may be placed on different locations, wherein each element may comprise or cooperate with a communication element configured for communication between the at least two elements of the apparatus. By way of example, the different locations may be in the same room, building, town, or country, or distributed over at least two continents. Further examples are conceivable.

As further generally used, the term "aerosol" refers to an aerosolizable material that comprises solid or liquid particles of a substance which are suspended in a gas phase, wherein the particles may, in particular, be or comprise particles of a pharmaceutical preparation, such as but not limited to a lung surfactant. For converting the particles into this state, an aerosolizable material, i.e. a powder or a liquid solution, is treated in an aerosol generating element, in particular by vibrating meshes or ultrasonic waves in order to entrain the solid or liquid particles into a gas stream of a carrier gas, such as a respiratory gas. In this state, the particles are, preferably, distributed across the entire volume of the carrier gas, in particular, in a uniform and finely dispersed form. As a result, the aerosol is provided as an "aerosol stream" in which the solid or liquid aerosol particles are borne or carried by the carrier gas stream. Further, the term "administering" or any grammatical variation thereof refers to a process of providing a controlled application of the respiratory gases and the aerosol comprised hereby, in particular, by carrying a predefined amount of the pharmaceutical preparation as comprised by the humidified aerosol per period of time to the patient. As used herein, the term "respiratory gases" refers to a gas mixture which comprises a composition being suitable for the ventilation of a patient, in particular, air or oxygen-enriched air.

Further, the term "ventilatory circuit" refers to a device configured for a ventilation of the respiratory gases as provided by a ventilator to the patient and from the patient back to the ventilator, hereby excluding the respiratory tracks of the patient. As further used herein, the term "patient" refers to a human being of any age, in particular, including a preterm infant. Further, the term "ventilation" relates to a process of accomplishing a movement of the respiratory gases, in particular, via alternating steps of inhalation and exhalation. In contrast to normally breathing patients who are capable of performing the circulation without any additional aids, patients who are subject to mechanical ventilation, require the respiratory gases at least partially to be provided from the ventilator via the ventilatory circuit. As used herein, the term "patient interface" refers to a device being configured for providing a connection between the ventilatory circuit and the respiratory track of the patient which is therefore, in general, located adjacent to the patient. For this purpose, the patient interface may be integrated into, or attached to, the ventilatory circuit, wherein the ventilatory circuit may, in general, comprise a ventilator and tubes adapted for guiding gases from the ventilator to a patient interface and back. In particular, a suitable mouthpiece, a breathing mask, a nasal cannula, a nasal prong or a tracheal cannula may be part of the patient interface or attachable thereto. However, further arrangements may also be feasible.

As used herein, the term "processing" or any grammatical variation thereof refers to applying at least one algorithm to data received by at least one input file in a manner that the desired controlling of the apparatus for administering the aerosol to the patient is provided by at least one output file which comprises at least one command for controlling an aerosol valve configured for providing the aerosol stream to the patient. With particular regard to the present invention, the term "data" refers to at least one piece of information comprised by at least one file, specifically by at least one input file or at least one output file. With particular respect to the present invention, the at least one piece of information comprised by at least one input file is related to a respiration pattern of the patient, while the at least one piece of information comprised by at least one output file is related to the at least one command for controlling the aerosol stream to the patient which is triggered at at least one point in time. Herein, the at least one algorithm may be configured to determine the data for the at least one output file from the data provided by the at least one input file according to a predefined scheme, wherein, as described below in more detail, artificial intelligence, in particular at least one machine learning algorithm, may also be applied.

As generally used, the term "processing device" refers to a particular kind of device which is designated for determining the data for the at least one output file from the data provided by the at least one input file, wherein the at least one input file may, preferably, be provided to the processing device by using at least one input interface, and wherein the at least one output file may, preferably, be provided by the processing device by using at least one output interface. Specifically, the processing device may comprise at least one of an integrated circuit, in particular an application-specific integrated circuit (ASIC), or a digital processing device, in particular at least one of a digital signal processor (DSP), a field programmable gate array (FPGA), a microcontroller, a microcomputer, a computer, or a mobile communication device, specifically a notebook, a tablet, a smartphone, or a personal digital assistant. Further components may be feasible, in particular at least one of a preprocessing element or a data storage element. The processing device may, preferably, be designed to perform at least one computer program, in particular at least one line of computer program code configured to execute at least one algorithm for determining the data for the at least one output file, wherein the processing of the data may be performed in at least one of a consecutive or a parallel manner.

According to the present invention, the processing device is configured for controlling the apparatus for administering the aerosol to the patient, firstly, by receiving input data that are related to a respiration pattern of a patient. As generally used, the term "receiving" or any grammatical variation thereof refers to a process of obtaining at least one piece of information, in particular as at least one input file. With particular regard to the present invention, the at least one input file comprises data which are related to a respiration pattern of the patient. As further generally used, the term "respiration pattern" relates to a temporal course of an alteration of at least one body part of a patient owing to and related to a breathing of the patient. Herein, each respiration pattern can be identified by a first point in time which is related to an onset of the breathing of the patient and by a second point in time which is related to a suspension of the breathing of the patient, wherein the first point in time and the second point in time arise in an alternating manner, thereby defining a respiratory cycle. In many cases, the respiration pattern has a regular pattern being repeated after each respiratory cycle, wherein, typically, each respiratory cycle may have approximately the same time duration. However, alternatively or in addition, the respiration pattern may also comprise at least one irregular feature with respect to the respiration of the patient. Especially, a preterm infant or a seriously ill adult may, however, have a difference in the time duration for consecutive respiration patterns. Advantageously, the present invention operates, as described below in more detail, independently of whether the respiration pattern may be a regular pattern or not.

Further, the processing device is configured for determining at least one point in time from the respiration pattern. As generally used, the term "determining" or any grammatical variation thereof refers to a process of generating representative results which are, typically, denoted as "data". With particular regard to the present invention, the data which are generated by the processing device corresponds to at least one output file comprising at least one piece of information that is related to at least one command, which is configured for controlling the aerosol stream to the patient that is triggered at the at least one point in time. For this purpose, the at least one command corresponds to at least one piece of information that is directed to altering a flow rate of the aerosol stream, in particular one of commencing, increasing, maintaining, decreasing, or terminating the volume of the aerosol stream. As described below in more detail, an aerosol valve configured for providing the aerosol stream to the patient can be controlled for this purpose, however, other procedures, such as altering the gas stream of the carrier gas, may, in principle, also be feasible.

Further, the processing device is configured for controlling the aerosol stream to the patient that is triggered at the at least one point in time. As generally used, the term "controlling" or any grammatical variation thereof refers to a process of adjusting at least one property of an object or a state by using at least one command. As further generally used, the term "triggering" or any grammatical variation thereof refers to a signal which is configured for commencing an alteration of the object or the state. With respect to the present invention, the aerosol stream to the patient is controlled in a manner that the flow rate of the aerosol stream is altered upon the signal being provided by the at least one command as determined by the processing device from the input data.

According to the present invention, the respiration pattern comprises information about the temporal course of at least one of an elevation or a constriction of at least one of a chest or an abdomen of a patient. As generally known, the chest and the abdomen of the patient experiences the elevation and the constriction in an alternating manner, in particular owing to the breathing of the patient, whereby the respiratory cycle is defined. As generally used, the term "elevation" refers to a rise of the abdominal wall and/or the rip cage, while the term "constriction" relates to a lowering of the abdominal wall and/or the rip cage, whereby they resume their preceding position. As indicated above, the respiratory cycle can be regular or not, wherein the present invention has a particular advantage in that it allows controlling the aerosol stream to the patient independently of whether the respiration pattern is regular or not.

Further, controlling the aerosol stream to the patient comprises a triggering of the aerosol stream at the at least one point in time, i.e. at at least one of the first point in time related to the onset of the breathing of the patient or the second point in time related to the suspension of the breathing of the patient, wherein a triggering of the aerosol stream at the first point in time may, generally, be preferred to the triggering of the aerosol stream at the second point in time. In particular accordance with the present invention, the at least one point in time advances at least one of the onset or the suspension of the breathing of the patient, wherein the onset and the suspension of the breathing of the patient may, in particular, correspond to the onset or the suspension of the aerosol stream through at least one of a nose or a mouth of the patient. As a consequence, while the first point in time is related to the onset of the breathing of the patient by being determined from the elevation of at least one of the chest or the abdomen of the patient, it, nevertheless, advances the onset of the breathing of the patient as indicated by an onset and a subsequent increase of a volume of the aerosol stream through at least one of the nose or the mouth of the patient. Similarly, while the second point in time is related to the suspension of the breathing of the patient by being determined from the constriction of at least one of the chest or the abdomen of the patient, it, nevertheless, advances the suspension of the breathing of the patient as indicated by a drop and a subsequent decrease of the volume of the aerosol stream through at least one of the nose or the mouth of the patient. In particular, the at least one point in time may advance the onset or the suspension of the breathing of the patient, respectively, by a time interval of 1 millisecond, preferably 50 milliseconds, more preferred of 100 ms, to 1 second, preferably 500 milliseconds, more preferred of 200 milliseconds; however, depending on the particular patient, a different value may also be feasible.

In other words, the aerosol stream to the patient may, preferably, be triggered by the at least one command as determined by the processing device from the input data which comprise information about the elevation or the constriction of at least one of the chest or the abdomen of the patient and, thereby, advances at least one of the onset or the suspension of the breathing of the patient in a favorable manner. It has been found that the input data which correspond to a movement of the chest or the abdomen of the patient are, particularly, suitable for determining the at least one point in time at which the triggering of the aerosol stream to the patient is released. While the movement of the nose and the mouth of the patient are synchronous with the breathing of the patient, the movement of the chest or the abdomen of the patient precede by the time interval as indicated above and can, therefore, be used for the purposes of the present invention, especially for administering a pharmaceutical preparation comprised by the aerosol to the patient, in particular to ensure that a predominant amount, preferably a complete amount, of the pharmaceutical preparation may actually be provided to the patient in a desired concentration and may not be diluted or distributed elsewhere.

This advantage is in particular contrast to the known prior art, according to which up to 90 vol.% or more of a pharmaceutical preparation may actually be distributed elsewhere apart from the patient, and according to which a, typically, high dilution of a pharmaceutical preparation during administering to the patient occurs. Rather, advancing the triggering of the aerosol by a small time interval with respect to the onset or the suspension of the breathing of the patient, respectively, results in a particular advantageous effect that the pharmaceutical preparation comprised by the aerosol is introduced into the respiratory gases at a point in time at which it can be ensured that the predominant amount, preferably the complete amount of the introduced pharmaceutical preparation may actually be received by the respiratory tracks of the patient, in particular at least one predefined target in at least one of a central or a peripheral area of the lung may be possible. Moreover, by near-patient breath-triggered release of the pharmaceutical preparation it may further be possible to reduce or, preferably, to avoid a delay in administering the pharmaceutical preparation to the patient. In this manner, an optimal breath-triggered release of the pharmaceutical preparation can be achieved, in particular by providing a cloud of aerosol to at least one of the nose or the mouth of the patient prior to the onset of inhalation flow. Such a kind of "pre-triggering" is provided by the present invention since it enables detecting an onset of the inhalation flow prior to measuring an onset of the respiratory flow. Similar results can also be achieved for the suspension of the respiratory flow by using the present invention. In addition, using the kind of input data according to the present invention, which have specifically been generated for this purpose, ensures that this effect is independent of whether the respiration pattern of the patient may be a regular pattern or not. As a result, the present invention can, especially, be employed for treating and curing preterm infants or seriously ill adults who, typically, exhibit an irregular respiration pattern.

In a further aspect, the present invention refers to an apparatus for administering an aerosol to a patient. Accordingly, the apparatus comprises:
- a processing device as disclosed herein; and
- an aerosol valve configured for providing the aerosol stream to the patient.

Accordingly, the apparatus for administering the aerosol to the patient comprises the processing device as described above and below in more detail and, in addition, an aerosol valve which is configured for providing the aerosol stream to the patient, wherein the processing device is configured for controlling the aerosol stream through the aerosol valve by triggering the aerosol valve at the at least one point in time, whereby the aerosol stream designated for being provided to the patient is adjusted in the manner as disclosed herein.

As generally used, the term "aerosol valve" refers to a device which is configured for controlling a volume of an aerosol stream, especially for being provided to at least one of the nose or the mouth of the patient. As indicated above, the aerosol valve can be controlled by at least one command comprising at least one piece of information which is directed to altering a flow rate of the aerosol stream, in particular one of commencing, increasing, maintaining, decreasing, or terminating the volume of the aerosol stream. For this purpose, a pneumatic device which can be addressed by the at least one command may be used; however a further embodiment may also be feasible. In a preferred embodiment, the aerosol valve can be integrated into the patient interface which is, as described above, configured for providing a connection between the ventilatory circuit and the respiratory track of the patient. As an alternative, the aerosol valve can be located in an upstream position with respect to the patient interface. Further alternatives are conceivable.

In a preferred embodiment of the present invention, the apparatus for administering the aerosol to the patient may, further, comprise a respiratory detection device. As used herein, the term "respiratory detection device" is a device which is configured for determining the input data related to the respiration pattern of the patient and for transmitting the input data related to the respiration pattern of the patient to the processing device as disclosed herein.

With particular respect to the present invention, the respiratory detection device may be configured for determining at least one of the elevation or the constriction of at least one of the chest or the abdomen of the patient, which are related to the respiration of the patient. In a particular embodiment, the respiratory detection device may at least comprise
- a detection element configured for recording at least one of the elevation or the constriction of at least one of the chest or the abdomen of the patient;
- a processing element configured for determining the input data related to the respiration pattern of the patient from at least one of the elevation or the constriction of the at least one of the chest or the abdomen of the patient; and
- a communication interface configured for transmitting the input data to the processing device.
Additional components are conceivable.

As used herein, the term "detection element" refers to device or a portion thereof, which is configured for recording and measuring at least one physical property related to a movement of a body part. Herein, the detection element may operate in contact with the body of the patient or, alternatively or in addition, in a contactless manner. In particular, the detection element may, be a breath detection sensor selected from at least one of a strain-gauge element, a time-of-flight camera, an electrical impedance tomography sensor, a respiratory inductive plethysmography sensor, a millimeter wave sensor, a radar sensor, or a thermal sensor. However, using a different type of detection element may also be feasible.

As further used herein, the term "processing element" refers to a further device or a portion thereof, which is configured for determining at least one piece of data. Herein, the processing element and the communication interface may, preferably, constitute a single device, while the detection element may constitute a separate device. An embodiment in which the processing element, the detection element and the communication interface may form an integrated device may also be feasible. With regard to the present invention, the processing element may, in particular, be configured for receiving measurement data about at least one of the elevation or the constriction of at least one of the chest or the abdomen of the patient from the detection element, wherefrom it may determine the input data related to the respiration pattern of the patient.

As further used herein, the term "communication interface" refers to a transmission channel which is designated for transmitting data from a first location to a second location, which, particularly, differs from the first location. Preferably, the communication interface may be a unidirectional interface configured for forwarding data into a single direction, such as from the processing element to the processing device. Alternatively, the communication interface may be a bidirectional interface configured for forwarding data into one of two directions, such as from the processing element to the processing device, or vice versa, in particular for further transmitting at least one command from the processing device to the processing element, wherein the at least one command may be selected from commencing or finishing a measurement, or commencing or finishing a data transmission. For a purpose of data transmission, the communication interface may comprise at least one of a wire-bound element or a wireless element, wherein the wireless element may be configured to operate by using a wireless communication protocol, such as Wi-Fi or Bluetooth; however, a further kind of communication interface may also be feasible. In a particular embodiment, the communication may be or comprise an encrypted data transfer or an encrypted data exchange, especially for a protection of personal data.

In a further preferred embodiment of the present invention, the apparatus for administering the aerosol to the patient may, further, comprise an aerosol generating device configured for generating the aerosol. In particular, the aerosol generating device may comprise an air inlet that may be configured for receiving a portion of breathing air from the respiratory circuit to be provided to an aerosol generating element as further comprised by the aerosol generating device. Herein, the aerosol generating element may, especially, be configured for generating aerosol particles and introducing them into the aerosol stream. As already indicated above, the term "aerosol generating element" refers to an element which is designated for converting an aerosolizable material, i.e. a powder or a liquid solution, into an aerosol, in particular by vibrating meshes or ultrasonic waves in order to entrain the solid or liquid particles into the gas stream of the carrier gas, such as the respiratory gas. For this purpose, the aerosol generating element may, preferably, be selected from at least one of a nebulizer or a powder generator. Further, the aerosol generating device may, in particular, comprise an aerosol tube which is configured for providing the aerosol stream that comprises the aerosol particles through a conducting element, in particular the aerosol valve, to the patient.

In a particular embodiment, the aerosol generating device may, further, comprise a ventilator which may be configured for providing an additional pressure within the aerosol stream. In particular, the additional pressure may be used for improving a manner of guiding of the aerosol stream to the patient through a conducting element, in particular the aerosol valve. Preferably, a pressure which may be prevailing at the aerosol tube, when the aerosol valve is in an open position, may exceed a further pressure that may be prevailing at the air inlet by at least 0.1 mbar, preferably by at least 0.05 mbar, more preferred by at least 0.02 mbar, especially by at least 0.01 mbar.

In an additionally preferred embodiment, the ventilator may, further, be configured for limiting the additional pressure within the aerosol generating device to an additional peak pressure. Herein, the additional peak pressure may assume a value of at maximum 10 mbar, preferably 5 mbar, more preferred 2 mbar, especially 1 mbar, or below. Limiting the additional peak pressure in the patient interface, when the aerosol valve is in an open position, can contribute to avoiding a damage to the patient by a too high pressure.

In a further particular embodiment, the aerosol generating device may, additionally, comprise a breathing filter that may be placed at an upstream location with respect to the aerosol generating element. As generally used, the term "breathing filter" refers to a filtering element which is configured for removing at least one interfering substance from the breathing air to be provided to the aerosol generating element.

For details concerning the apparatus for administering the aerosol to the patient, reference can be made to the description of the processing device and of the exemplary embodiments elsewhere in this document.

In a further aspect, the present invention refers to an aerosol generating device configured for generating the aerosol. This particular aspect refers to an embodiment of the present invention which solely comprises the aerosol generating device as disclosed herein, especially without encompassing the processing device. Accordingly, the aerosol generating device comprises:
- an air inlet configured for receiving a portion of breathing air from a ventilatory circuit to be provided to an aerosol generating element;
- the aerosol generating element configured for generating aerosol particles and introducing them into the aerosol stream;
- an aerosol tube configured for providing the aerosol stream comprising the aerosol particles through a conducting element to the patient.
Herein, the conducting element may, preferably be the aerosol valve as disclosed herein. In addition, the aerosol generating device may comprise at least one further element, in particular at least one of a ventilator or a breathing filter as disclosed elsewhere herein. For details concerning the aerosol generating device, reference can be made to the description of the apparatus for administering the aerosol to the patient and of the exemplary embodiments in this document.

In a further aspect, a method for administering an aerosol to a patient is proposed, wherein the method comprises the following steps a) to c):
a) receiving input data related to a respiration pattern of a patient, wherein the respiration pattern comprises information about a temporal course of at least one of an elevation or a constriction of at least one of a chest or an abdomen of a patient;
b) determining at least one point in time from the respiration pattern; and
c) controlling an aerosol stream to the patient triggered at the at least one point in time,
wherein the at least one point in time advances at least one of an onset or a suspension of a breathing of the patient.

Herein, the indicated steps a) to c) may be performed in the order as provided, wherein, preferably, all of the indicated steps may be preformed at least partially concurrently. Further, additional method steps, whether described in this document or not, can be performed, too.

The method for administering an aerosol to a patient as disclosed herein may, preferably, be a computer-implemented method. As generally used, the term "computer-implemented method" refers to a method involving at least one programmable device, particularly, selected from a mobile communication device. However, a further kind of programmable device, such as may also be feasible. Herein, the at least one programmable device may, especially, comprise the processing device as disclosed herein or have access thereto, wherein at least one feature of the method is performed by using at least one computer program. For this purpose, the computer program may be provided on the at least one programmable device, or the at least one programmable device may have access to the computer program via a network, such as an in-house network or the internet, which may be located in a remote server or a cloud.

According to step a), the input data which are related to the respiration pattern of the patient are received, wherein the respiration pattern comprises information about the temporal course of at least one of the elevation or the constriction of at least one of the chest or the abdomen of the patient.

According to step b), at least one point in time is determined from the respiration pattern as described elsewhere herein in more detail.

According to step c), the aerosol stream to the patient is controlled by triggering the aerosol stream at the at least one point in time, wherein the at least one point in time advances the at least one of the onset or the suspension of the breathing of the patient.

For details concerning the method for administering the aerosol to the patient, reference can be made to the description of the processing device, the apparatus for administering an aerosol to a patient, and the exemplary embodiments elsewhere in this document.

As used herein, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

### Short description of the Figures

Further optional features and embodiments of the invention will be disclosed in more detail in the subsequent description of preferred embodiments, preferably in conjunction with the dependent claims. Therein, the respective optional features may be implemented in an isolated fashion as well as in any arbitrary feasible combination, as the skilled person will realize. It is emphasized that the scope of the invention may not be restricted by the preferred embodiments. The embodiments are schematically depicted in the Figures. Therein, identical reference numbers in these Figures refer to identical or functionally comparable elements.

In the Figures:
- Figure 1: schematically illustrates a preferred embodiment of an exemplary apparatus for administering an aerosol to a patient according to the present invention;
- Figure 2: schematically illustrates a comparison between temporal courses of first signals generated by using a strain-gauge element and of second signals generated by using a flow sensor during an exhalation phase (Fig. 2A) and an inhalation phase (Fig. 2 B), respectively;
- Figure 3: schematically illustrates schematically illustrates respiratory phases of a preterm infant extracted from an abdominal motion data recorded with a time-of-flight camera; and
- Figure 4: schematically illustrates a comparison of a dose efficiency of a pharmaceutical preparation for various tests with respect to an emitted dose.

### Detailed description of the embodiments

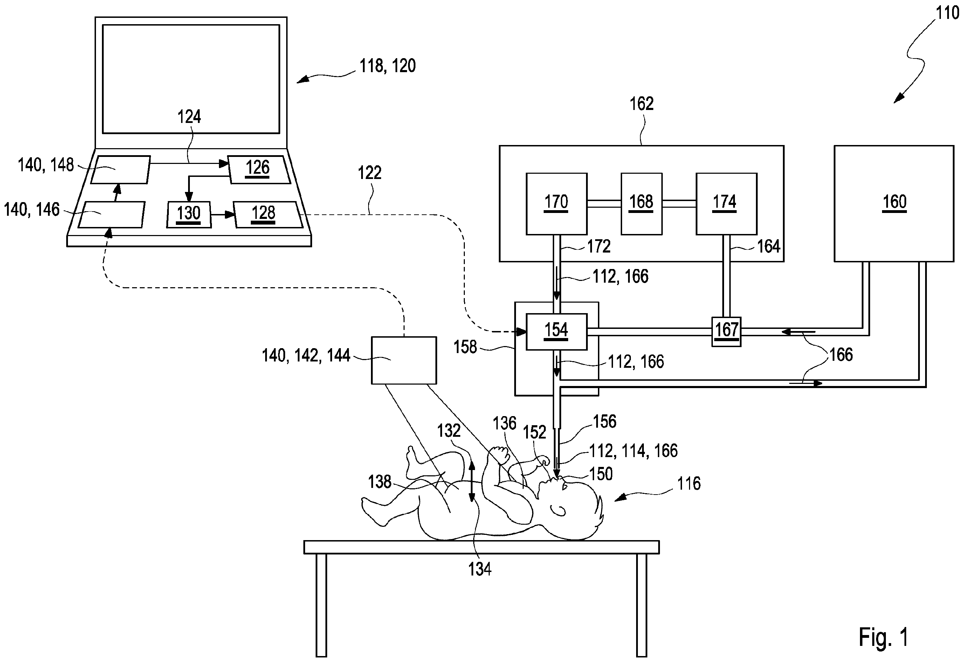

Figure 1 schematically illustrates a preferred embodiment of an exemplary apparatus 110 for administering an aerosol 112, in particular in form of an aerosol stream 114, to a patient 116. In particular, the apparatus 110 designed for providing the aerosol stream 114 having a defined aerosol concentration to the patient 116, who may, especially, be a preterm infant or a seriously ill adult. However, further applications of the apparatus 110 are feasible.

Further, the exemplary apparatus 110 as depicted in Figure 1 comprises a notebook 118, which is or comprises a processing device 120 configured for controlling the apparatus 110 for administering the aerosol 112, preferably in form of the aerosol stream 114, to the patient 116. As an alternative, the processing device 120 may be or be comprised by a different kind of mobile communication device, especially a tablet, a smartphone, or a personal digital assistant. As a further alternative, at least one of an integrated circuit, in particular an application-specific integrated circuit (ASIC), or a digital processing device, in particular at least one of a digital signal processor (DSP), a field programmable gate array (FPGA), a microcontroller, a microcomputer, or a computer can be used as the processing device 120.

The processing device 120 is designated for determining data for at least one output file 122 from data provided by the at least one input file 124, wherein the at least one input file 124 may be provided to the processing device 120 by using an input interface 126, and wherein the at least one output file 122 may be provided by the processing device 120 by using an output interface 128. The processing device 120 as shown in Figure 1 is further designed to perform at least one computer program, in particular at least one line of computer program code, configured to execute at least one algorithm 130 for determining the data for the at least one output file 122, wherein the processing of the data can be performed by using the at least one algorithm 130 in a consecutive and/or a parallel manner.

For this purpose, the processing device 120 is configured for receiving the at least one input file 124 comprising input data related to a respiration pattern of the patient 116. The respiration pattern in this exemplary embodiment comprises information about a temporal course of an elevation 132 and a constriction 134 of a chest 136 or an abdomen 138 of the patient 116. In this manner, information about a rise and a lowering of an abdominal wall and a rip cage of the patent 116 can be obtained, wherefrom the respiration pattern can be derived. In general, the respiration pattern may be a regular respiration pattern or, especially for a preterm infant or a seriously ill adult, an irregular respiration pattern, wherein the present invention has a particular advantage in that it allows controlling the aerosol stream 114 to the patient 116 independently of whether the respiration pattern may be regular or not.

The exemplary apparatus 110 as depicted in Figure 1, further, comprises a respiratory detection device 140, which is configured for determining the at least one input file 124 comprising the input data related to the respiration pattern of the patient 116 and for transmitting the at least one input file 124 to the processing device 120 by using the input interface 126. With particular respect to the present invention, the respiratory detection device 140 is configured in this exemplary embodiment for determining both the elevation 132 and the constriction 134 of the chest 136 and/or the abdomen 138 of the patient 116, which are related to the respiration of the patient 116.

As further illustrated in Figure 1, the respiratory detection device 140 comprises a detection element 142, which is configured in this exemplary embodiment for recording both the elevation 132 and the constriction 134 of the chest 136 and/or the abdomen 138 of the patient 116. In general, the detection element 142 may operate in contact with the body of the patient 116 or, alternatively or in addition, in a contactless manner. As depicted there, the detection element 142 is or comprises a time-of-flight camera 144, which is configured for recording and measuring at least one physical property related to a movement of the chest 136 and/or the abdomen 138 of the patient 116 in a contactless manner. As an alternative, a different type of a breath detection sensor can be used as the detection element 142, especially a contact-providing element, such as a strain-gauge element, or a different contactless element, such as an electrical impedance tomography sensor, a respiratory inductive plethysmography sensor, a millimeter wave sensor, a radar sensor, or a thermal sensor.

As further illustrated in Figure 1, the respiratory detection device 140 further comprises the notebook 118 which, additionally, has a processing element 146, which is configured for determining the at least one input file 124 comprising the input data related to the respiration pattern of the patient 116, and a communication interface 148, which is configured for transmitting the at least one input file 124 comprising the input data related to the respiration pattern of the patient 116 to the input interface 126 comprised by processing device 120. In the exemplary embodiment of Figure 1, the processing element 146 and the communication interface 148 are both comprised by the notebook 118, while the detection element 142 is embodied as a separate device. However, other embodiments are feasible, in particular an embodiment in which the detection element 142, the processing element 146 and the communication interface 148 may form an integrated device (not depicted here). For a purpose of data transmission, the communication interface 148 may comprise at least one of a wire-bound element or a wireless element configured to operate by using a wireless communication protocol, such as Wi-Fi or Bluetooth, wherein an encrypted data transfer or an encrypted data exchange, especially for a protection of personal data, may also be feasible.

The communication interface 148 may be or comprise a unidirectional interface configured for transmitting data into a single direction from the processing element 146 to the input interface 126 comprised by processing device 120. Alternatively, the communication interface 148 may be a bidirectional interface configured for forwarding data into one of two directions, such as from the from the processing element 146 to the input interface 126, or vice versa, in particular for further transmitting at least one command from the processing device 120 to the processing element 146, wherein the at least one command may be directed to commencing or finishing a measurement, or commencing or finishing a data transmission.

Further, the processing device 120 is configured for determining at least one point in time from the respiration pattern and for controlling the aerosol stream 114 to the patient 116 triggered at the at least one point in time, wherein the at least one point in time advances an onset and/or a suspension, respectively, of a breathing of the patient. In particular, the at least one point in time may advance the onset or the suspension, respectively, of the aerosol stream 114 through a nose 150 or a mouth 152 of the patient 116. More particular, the at least one point in time may advance the onset or the suspension, respectively, of the breathing of the patient 116 by a time interval of 1 millisecond, preferably 50 milliseconds, more preferred of 100 ms, to 1 second, preferably 500 milliseconds, more preferred of 200 milliseconds; however, depending on the particular patient 116, a different value may also be feasible. In this respect, the aerosol stream 114 to the patient 116 is triggered by at least one command as comprised by the at least one output file 122 as determined by the processing device 120 by using the at least one algorithm 130, wherein the at least one output file 122 comprises at least one command being forwarded by using an output interface 128 to an aerosol valve 154 being configured for providing the aerosol stream 114 to the patient 116.

It has been found that using the at least one input file 124 comprising the input data related to the respiration pattern of the patient 116 as determined by using the information about the elevation 132 and/or the constriction 134 of the chest 136 and/or the abdomen 138 of the patient 116 allows using the processing device 120 for predicting the at least one point in time at which the triggering of the aerosol stream 114 to the patient 114 can reasonably be released. In contrast to the movement of the nose 152 and the mouth 152 of the patient 116, which are synchronous with the breathing of the patient 116, the movement of the chest 136 and/or the abdomen 138 of the patient 114 precede by the time interval as indicated above, which can, therefore, be used for the purposes of the present invention, especially for administering a pharmaceutical preparation comprised by the aerosol 112 to the patient 116. In this manner, it can be ensured that a predominant amount, preferably a complete amount, of the pharmaceutical preparation may actually be provided to the patient 116 and not distributed elsewhere, such as to an undesired portion of the apparatus 110 or to surroundings of the apparatus 110. By using the at least one input file 124 it can, further be ensured that this effect is independent of whether the respiration pattern of the patient may be regular or not.

In further accordance with the present invention, the apparatus 110 further comprises the aerosol valve 154, which is configured for providing the aerosol stream 114 to the patient 116. For this purpose, the aerosol valve 154 is configured for controlling a volume of the aerosol stream 114 which is, particularly, provided to the nose 150, such as by using a pair of nasal prongs 156, or to the mouth 152 of the patient 116. As indicated above, the aerosol valve 154 can be controlled by the at least one command comprising at least one piece of information being directed to altering a flow rate of the aerosol stream 114, in particular one of commencing, increasing, maintaining, decreasing, or terminating the volume of the aerosol stream 114. In the exemplary embodiment of Figure 1, the aerosol valve 154 is integrated into a patient interface 158 configured for providing a connection between a ventilatory circuit 160 and a respiratory track of the patient. As an alternative (not depicted here), the aerosol valve 154 can be located at a different position, such as upstream with respect to the patient interface 158.

As further illustrated Figure 1, the exemplary embodiment of the apparatus 110 comprises an aerosol generating device 162, which is configured for generating the aerosol 114. In particular, the aerosol generating device 162 as depicted there comprise an air inlet 164 configured for receiving a portion of breathing air 166 via a junction 167 from the ventilatory circuit 160, which is provided here to a breathing filter 168 configured for removing at least one interfering substance from the breathing air 166 before being providing to an aerosol generating element 170 as further comprised by the aerosol generating device 162. Herein, the aerosol generating element 170 may, especially, be configured for generating aerosol particles and introducing them into the aerosol stream 114. For this purpose, the aerosol generating element 170 may be designated for converting an aerosolizable material, i.e. a powder or a liquid solution, into the desired aerosol 116, in particular by vibrating meshes or ultrasonic waves in order to entrain the solid or liquid particles into the gas stream that may comprise the breathing air 166 and a carrier gas, such as a respiratory gas. Herein, the aerosol generating element 170 may, preferably, be selected from a nebulizer and/or a powder generator. As further depicted in Figure 1, the aerosol generating device 162 further comprises an aerosol tube 172 which is configured for providing the aerosol 112 comprising the aerosol particles through a conducting element, in particular the aerosol valve 154, to the patient 116.

In the particular embodiment as illustrated in Figure 1, the aerosol generating device 162, further, comprises a ventilator 174, which is configured for providing an additional pressure within the aerosol stream 114. In particular, the additional pressure may be used for improving a manner of guiding of the aerosol stream 114 through the aerosol valve 154 to the patient 116. Preferably, a pressure prevailing at the aerosol tube 172, when the aerosol valve 154 is in an open position, may exceed a further pressure prevailing at the air inlet 164 by at least 0.1 mbar, preferably by at least 0.05 mbar, more preferred by at least 0.02 mbar, especially by at least 0.01 mbar. Preferably, the ventilator 174 may, further, be configured for limiting the additional pressure within the aerosol generating device to an additional peak pressure, wherein, the additional peak pressure may assume a value of at maximum 10 mbar, preferably 5 mbar, more preferred 2 mbar, especially 1 mbar, or below. Limiting the additional peak pressure in the patient interface 158, when the aerosol valve 154 is in an open position, can contribute to avoiding damaging the patient 116 by a too high pressure.

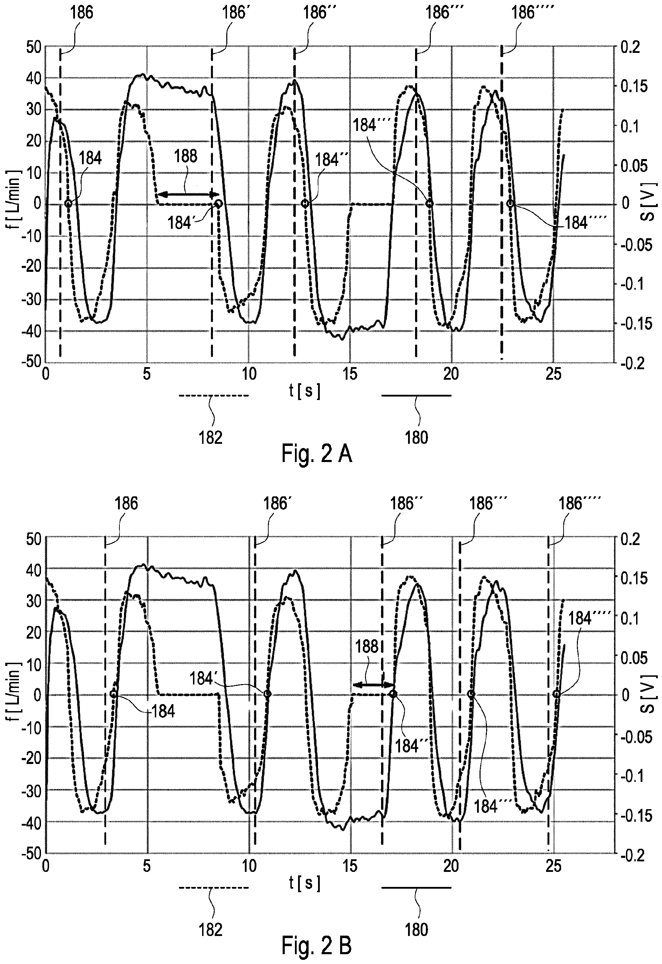

Figure 2 schematically illustrates a comparison between a first temporal course 180 of first signals generated by using a strain-gauge element and a second temporal course 182 of second signals generated by using a flow sensor during an exhalation phase (Fig. 2A) and an inhalation phase (Fig. 2B), respectively. Herein, the x-axis shows a time t in seconds s, the left y-axis a respiratory flow *f* as measured by using the flow sensor in *L*/*min,* and the right y-axis a mean signal S generated by the strain-gauge element in *volts.* A respective switch from exhalation to inhalation and vice versa is indicated by circles 184, 184', 184", . for the flow sensor and by vertical dashed lines 186, 186', 186", ... for the strain-gauge element. As indicated by a horizontal line 188, breathing was stopped twice in order to be able to accurately represent the onset of the inhalation and of the exhalation, respectively. A change from inhalation to exhalation is indicated by a zero crossing of the y-axis for the flow sensor and by extreme values or an abrupt slope increase or slope decrease, respectively, for the strain-gauge element. As shown in Figure 2, a maximum inhalation is followed by a maximum abdominal stretch, resulting in a maximum stretch of the strain-gauge element and, thus, a maximum positive tension. Similarly, a maximum exhalation is followed by minimum abdominal stretch, resulting in a minimum stretch of the strain-gauge element and, thus, a maximum negative stress. As shown in Figure 2, the strain-gauge element is capable of detecting the inhalation or the exhalation, respectively, for a time interval of about 200 ms to 300 ms earlier than the flow sensor. This time interval, by which the strain-gauge element is capable of detecting the oncoming inhalation or the exhalation earlier compared to the flow sensor, allows advancing the triggering of the aerosol 112 with respect to the onset or the suspension of the breathing of the patient 116, respectively, thus resulting in the advantageous effects described elsewhere herein.

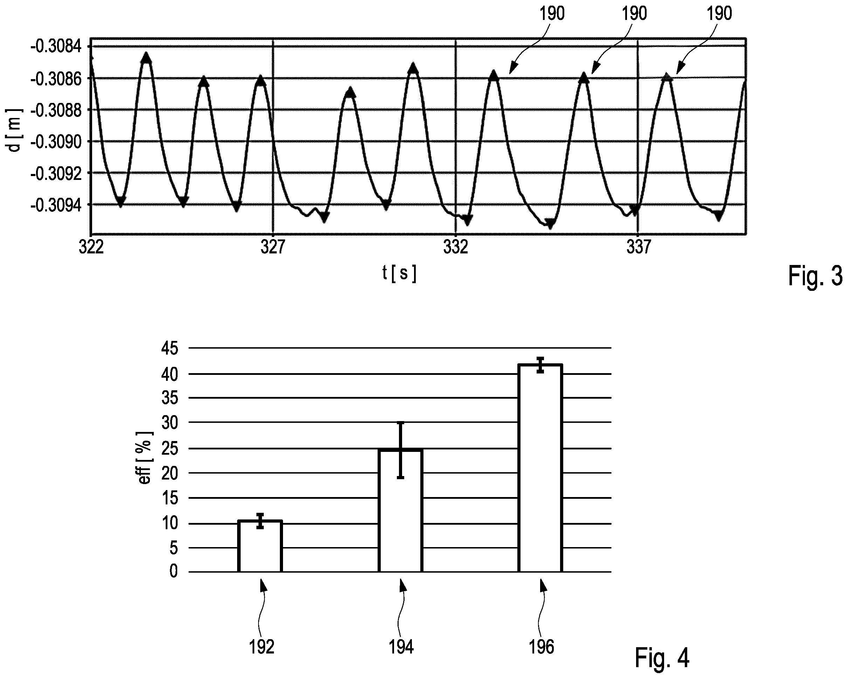

Figure 3 schematically illustrates respiratory phases 190 of a preterm infant extracted from an abdominal motion data recorded with a time-of-flight camera 144. Herein, a distance *d* in meters m from the time-of-flight camera 144to the preterm infant's abdomen 138 is plotted versus time t in seconds s. The maxima and minima as depicted in Figure 3 represent transitions from inhalation to exhalation and vice versa.

Figure 4 schematically illustrates a comparison of a dose efficiency *eff* of a pharmaceutical preparation for various tests 192, 194, 196 in % with respect to an emitted dose. In a first test 192, a standard patient interface according to the prior art was used, in a second test 194 the aerosol generating device 162 as disclosed herein was used, while and in a third test 196 the apparatus 110 according to the present invention including the aerosol generating device 162 as disclosed herein was used.

For this purpose, a 0.9 vol.% solution of saline was aerosolized using a mesh nebulizer and delivered to a respective test stand according to the above-mentioned tests 192, 194, 196 as follows:
- when using the using a standard patient interface in the first test 192, the aerosol was coupled into the ventilatory circuit 160 by using a Y-connector;
- when using the aerosol generating device 162 in the second test 194, the aerosol 112 was delivered directly into the patient interface 158 at a flow of approx. 1 *L*/*min.* The ventilatory circuit 160 was operated in continuous positive airway pressure (CPAP) mode using a positive end-expiratory pressure (PEEP) of 5 mbar and a respiratory gas flow of 6 *L*/*min.* For determining the delivered amount of aerosol 112, an advanced test rig as disclosed in WO 2020/007858 A1 was used. This test rig allows using different respiratory rates (30-80 breaths/min), tidal volumes (2-40 ml) and inhalation fractions (0.25-0.75). For the second test 194, a frequency of 51 breaths/min, a tidal volume of 12.3 ml, and an inspiratory fraction of 0.39 were used as simulated respiratory parameters. In this non-triggered mode of operation, the aerosol valve was 154 was open for the entire measurement period; and
- when using the apparatus 110 according to the present invention in the third test 196, the same conditions were applied as in the second test 194, however, the aerosol valve 154 was opened in the triggered mode of operation at the beginning of a simulated inspiration and closed at the beginning of a simulated expiration. For this purpose, the aerosol valve 154 was closed or opened by using a pneumatic device.

According to the results as depicted in Figure 4, the dose efficiency *eff* achieved in the first test 192 assumed a value of 10.3%, in the second test 194 of 24.7% (non-triggered release) and in the third test 196 of 41.5% (triggered release), respectively. Consequently, applying the non-triggered release by using the aerosol generating device 162 is more efficient than using the standard patient interface by a factor of approx. 2.5. Further, using the breath-triggered release in combination with the aerosol generating device 162 is more efficient by a factor of approx. 1.7 compared to the non-triggered release and by a factor of approx. 4.2 with respect to using the standard patient interface.

### List of reference numbers

- 110: apparatus
- 112: aerosol
- 114: aerosol stream
- 116: patient
- 118: notebook
- 120: processing device
- 122: output file
- 124: input file
- 126: input interface
- 128: output interface
- 130: algorithm
- 132: elevation
- 134: constriction
- 136: chest
- 138: abdomen
- 140: respiratory detection device
- 142: detection element
- 144: time-of-flight camera
- 146: processing element
- 148: communication interface
- 150: nose
- 152: mouth
- 154: aerosol valve
- 156: nasal prong
- 158: patient interface
- 160: ventilatory circuit
- 162: aerosol generating device
- 164: air inlet
- 166: breathing air
- 167: junction
- 168: breathing filter
- 170: aerosol generating element
- 172: aerosol tube
- 174: ventilator
- 180: first temporal course
- 182: second temporal course
- 184, 184', ....: circle
- 186, 186", ...: vertical dashed line
- 188: horizontal line
- 190: respiratory phase
- 192: first test
- 194: second test
- 196: third test

## Claims

1. A processing device (120) configured for controlling an apparatus (110) for administering an aerosol (112) to a patient (116) by
- receiving input data related to a respiration pattern of a patient (116), wherein the respiration pattern comprises information about a temporal course of at least one of an elevation (132) or a constriction (134) of at least one of a chest (136) or an abdomen (138) of a patient (116);
- determining at least one point in time from the respiration pattern; and
- controlling an aerosol stream (114) to the patient (116) triggered at the at least one point in time,
wherein the at least one point in time advances at least one of an onset or a suspension of a breathing of the patient (116).

2. The processing device (120) according to the preceding claim, wherein the onset and the suspension of the breathing of the patient (116) corresponds to the onset or the suspension of the aerosol stream (114) through at least one of a nose (150) or a mouth (152) of the patient (116).

3. The processing device (120) according to any one of the preceding claims, wherein the at least one point in time advances the at least one of the onset or the suspension of the breathing of the patient (116) by a time interval of 1 millisecond to 1 second.

4. An apparatus (110) for administering an aerosol (112) to a patient (116), comprising
- a processing device (120) according to any one of the preceding claims; and
- an aerosol valve (154) configured for providing the aerosol stream (114) to the patient (116).

5. The apparatus (110) according to the preceding claim, further comprising an aerosol generating device (162) configured for generating the aerosol stream (114).

6. The apparatus (110) according to the preceding claim, wherein the aerosol generating device (162) comprises
- an air inlet (164) configured for receiving a portion of breathing air (166) from a ventilatory circuit (160) to be provided to an aerosol generating element (170);
- the aerosol generating element (170) configured for generating aerosol particles and introducing them into the aerosol stream (114);
- an aerosol tube (172) configured for providing the aerosol stream (114) comprising the aerosol particles through a conducting element, in particular the aerosol valve (154), to the patient (116).

7. The apparatus (110) according to the preceding claim, wherein the aerosol generating element (170) is selected from at least one of a nebulizer or a powder generator.

8. The apparatus (110) according to any one of the three preceding claims, wherein the aerosol generating device (162) further comprises a ventilator (174) configured for providing an additional pressure within the aerosol stream (114) for guiding the aerosol stream (114) through the aerosol valve (154) to the patient (116).

9. The apparatus (110) according to the preceding claim, wherein, when the aerosol valve (154) is in an open position, a pressure prevailing at the aerosol tube (172) exceeds a further pressure prevailing at the air inlet (164) by at least 0.01 mbar.

10. The apparatus (110) according to any one of the two preceding claims, wherein the ventilator (174) is further configured for limiting the additional pressure within the aerosol generating device (162) to an additional peak pressure.

11. The apparatus (110) according to any one of the six preceding claims, wherein the aerosol generating device (162) further comprises a breathing filter (168) upstream of the aerosol generating element (170), wherein the breathing filter (168) is configured for removing at least one interfering substance from the breathing air (166) to be provided to the aerosol generating element (170).

12. The apparatus (110) according to any one of the preceding apparatus claims, further comprising a respiratory detection device (140), wherein the respiratory detection device (140) is configured for determining at least one of the elevation (132) or the constriction (134) of the at least one of the chest (136) or the abdomen (138) of the patient (116) related to the respiration of the patient (116).

13. The apparatus (110) according to the preceding claim, wherein the respiratory detection device (140) comprises
- a detection element (142) configured for recording at least one of the elevation (132) or the constriction (134) of the at least one of the chest (136) or the abdomen (138) of the patient (116);
- a processing element (146) configured for determining the input data related to the respiration pattern of the patient (116) from the at least one of the elevation (132) or the constriction (134) of the at least one of the chest (136) or the abdomen (138) of the patient (116); and
- a communication interface (148) configured for transmitting the input data to the processing device (120).

14. The apparatus (110) according to the preceding claim, wherein the detection element (140) is a breath detection sensor selected from at least one of a time-of-flight camera (144), a strain-gauge element, an electrical impedance tomography sensor, a respiratory inductive plethysmography sensor, a millimeter wave sensor, a radar sensor, or a thermal sensor.

15. A method for administering an aerosol (112) to a patient (116), the method comprising the following steps:
a) receiving input data related to a respiration pattern of a patient (116), wherein the respiration pattern comprises information about a temporal course of at least one of an elevation (132) or a constriction (134) of at least one of a chest (136) or an abdomen (138) of a patient (116);
b) determining at least one point in time from the respiration pattern; and
c) controlling an aerosol stream (114) to the patient (116) triggered at the at least one point in time,
wherein the at least one point in time advances at least one of an onset or a suspension of a breathing of the patient (116).
